# EUROPÄISCHE PATENTSCHRIFT

(11) **EP 0 489 773 B1**
(45) Veröffentlichungstag und Bekanntmachung des Hinweises auf die Patenterteilung: **20.10.1993**
(21) Anmeldenummer: 90912639.3
(22) Anmeldetag: 28.08.1990
(51) Int. Cl.: C07B 63/00, C07C 17/38, C07C 23/36, C07C 23/38, C07D 265/30, C07D 211/38, C07D 207/10, C07D 401/06

(54) **VERFAHREN ZUR AUFTRENNUNG VON GEMISCHEN PARTIELL FLUORIERTER ODER PERFLUORIERTER KOHLENWASSERSTOFFVERBINDUNGEN**
PROCESS FOR SEPARATING MIXTURES OF PARTIALLY FLUORINATED OR PERFLUORINATED HYDROCARBON COMPOUNDS
PROCEDE POUR SEPARER DES MELANGES DE COMPOSES D'HYDROCARBURES PARTIELLEMENT FLUORES OU PERFLUORES

(30) Priorität: 30.08.1989 DE 3928692
(43) Veröffentlichungstag der Anmeldung: 17.06.1992
(73) Patentinhaber: Kali-Chemie Aktiengesellschaft, D-30173 Hannover (DE)
(72) Erfinder: MEINERT, Hasso, D-7900 Ulm (DE); MADER, Jürgen, D-7900 Ulm (DE)
(74) Vertreter: Lauer, Dieter, Dr.
(86) Internationale Anmeldenummer: DE9000658
(87) Internationale Veröffentlichungsnummer: WO9103442

(56) Entgegenhaltungen:
- DD-A- 256 692
- DE-B- 1 178 066
- DE-B- 1 206 424

## Beschreibung

Die vorliegende Erfindung betrifft ein Verfahren zur Auftrennung von Gemischen perfluorierter oder partiell fluorierter Kohlenwasserstoffverbindungen, insbesondere Perfluorkohlenwasserstoff-Gemischen, wie sie bei der elektrochemischen Perfluorierung von cyclischen Kohlenwasserstoffen erhalten werden.

Aus der DD-Patentschrift 256 629 ist bekannt, daß fluorierte Kohlenwasserstoffe zur Abtrennung von ihrer Umgebung an poröse Adsorbentien, unter anderem zeolithischen Molekularsieben, adsorbiert werden und aus den Poren durch übliche Verfahren zurückgewonnen werden können.

Die sogenannten Perfluorkohlenwasserstoffe (= Perfluorcarbone) sind perfluorierte organische Verbindungen, welche aus Kohlenstoff und Fluor bestehen und gegebenenfalls noch Heteroatome, zum Beispiel Stickstoff oder Sauerstoff, enthalten. Perfluorcarbone sind wasserunlösliche chemisch und biologisch inerte Verbindungen, welche bei Raumtemperatur unter Normaldruck flüssige bis wachsartige Konsistenz haben können. Derartige Verbindungen sind bekannt zum Beispiel aus den Europäischen Patentanmeldungen, Veröffentlichungsnummer 77 114, 99 652 und 151 697. Die Perfluorcarbonmoleküle sind durch eine einheitliche Schale von Fluoratomen hervorragend abgeschirmt. Daher sind Perfluorcarbone chemisch und physiologisch außerordentlich inert, also ungiftig. Wegen ihrer extrem geringen intermolekularen Kräfte haben Perfluorcarbone einen im Verhältnis zu ihren Molekülmassen tiefen Siedepunkt und eine außerordentlich geringe Oberflächenspannung. Aus den sehr schwachen intermolekularen Kräften resultiert auch die Fähigkeit der Perfluorcarbone, große Mengen von Gasen, beispielsweise Sauerstoff oder Kohlendioxid zu lösen. Aufgrund dieser Eigenschaften, insbesondere der Fähigkeit Sauerstoff physikalisch zu lösen und zu transportieren, haben Perfluorcarbone in der Medizin Anwendung gefunden zur Herstellung von wäßrigen sauerstofftransportierenden Perfluorcarbonemulsionen, welche zum Beispiel als Blutersatzmittel oder Perfusionsmedien eingesetzt werden können. Ferner sind Perfluorcarbone auch zur Anwendung in anderen technischen Gebieten, in welchen ungiftige und chemisch inerte flüssige oder wachsartige Substanzen benötigt werden, bzw. inerte Substanzen mit einem Lösevermögen für Gase benötigt werden, geeignet. So eignen sich Perfluorcarbone und ihre Gemische beispielsweise als inerte Kühlmittel, Schmiermittel, Sperr- und Hydraulikflüssigkeiten, Isoliermedien in der Elektrotechnik, sowie Mittel zum Dampfphasenlöten bzw. als Zusätze zu Mitteln für die vorgenannten Zwecke.

Perfluorierte oder partiell fluorierte Kohlenwasserstoffverbindungen werden im allgemeinen auf an sich bekannte Weise durch Fluorierung, beispielsweise elektrochemische Fluorierung, von entsprechenden nicht fluorierten Kohlenwasserstoffverbindungen erhalten. Bei der Fluorierung von cyclischen Ausgangsverbindungen werden oftmals Gemische erhalten, welche neben den partiell- oder perfluorierten cyclischen Produkten noch ebenfalls partiell fluorierte Nebenprodukte oder perfluorierte und somit chemisch und physiologisch inerte Nebenprodukte enthalten, welche acyclische Strukturelemente besitzen.Insbesondere können Fluorierungsprodukte cyclischer Auzgangsverbindungen solche perfluorierten Nebenprodukte enthalten, welche ein ähnliches Molekulargewicht wie das Hauptprodukt besitzen und damit im gleichen Temperaturbereich wie das Hauptprodukt sieden, beispielsweise acyclische Strukturelemente enthaltende Isomere des Hauptproduktes. Solche Gemische lassen sich durch fraktionierte Destillation nicht befriedigend auftrennen. Für viele Anwendungszwecke können die Gemische als solche eingesetzt werden, da die Anwesenheit perfluorierter Nebenprodukte die Anwendung nicht beeinträchtigt. In anderen Anwendungsgebieten, z.B. in der Medizin, werden jedoch reine Verbindungen gefordert.

Aufgabe der vorliegenden Erfindung ist es, ein einfaches zur Auftrennung von Gemischen perfluorierter oder partiell fluorierter Kohlenwasserstoffverbindungen, welche cyclische Verbindungen und Verbindungen mit acyclischen Strukturelementen enthalten, einsetzbares Trennverfahren zu finden.

Gegenstand der vorliegenden Erfindung ist ein Verfahren zur Auftrennung von Gemischen partiell- oder perfluorierter Kohlenwasserstoffverbindungen, welche gegebenenfalls noch Kettenglieder aus der Gruppe der Heteroatome Stickstoff, Sauerstoff, Bor, Phosphor und Schwefel enthalten und/oder durch weitere Halogenatome substituiert sein können, dadurch gekennzeichnet, daß man solche partiell- oder perfluorierten cyclischen Verbindungen, welche durch Reste mit unverzweigter acyclischer Struktur und mit einem kritischen Moleküldurchmesser im Bereich von 4 bis 7 Angström substituiert sind und welche nachfolgend als Verbindungen A bezeichnet werden, von solchen partiell- oder perfluorierten cyclischen Verbindungen, deren kritischer Moleküldurchmesser mindestens das 1,1-fache desjenigen der vorgenannten unverzweigten acyclischen Reste beträgt, und welche keine unverzweigten acyclischen Reste besitzen und auch nicht durch andere Reste substituiert sind, deren kritischer Moleküldurchmesser nicht mindestens das 1,1-fache desjenigen der vorgenannten unverzweigten acyclischen Reste beträgt, und welche nachfolgend als Verbindungen B bezeichnet werden, in flüssiger oder gasförmiger Phase trennt, indem man
a) das Gemisch oder eine Lösung des Gemisches in einem inerten organischen Lösungsmittel, dessen Molekulardurchmesser größer als derjenige der vorgenannten unverzweigten acyclischen Reste ist, mit einer zur Adsorption der Verbindungen A ausreichenden Menge eines Molekularsiebs kontaktiert, dessen mittlerer Porenöffnungsdurchmesser im Bereich des kritischen Moleküldurchmessers der unverzweigten
   acyclischen Reste der Verbindungen A oder bis zu 30 % darunter liegt,
b) das überwiegend mit Verbindungen A beladene Molekularsieb von der an Verbindungen A abgereicherten, die Verbindungen B enthaltenden flüssigen oder gasförmigen Phase abtrennt, und
c) die an dem Molekularsieb adsorbierten Verbindungen desorbiert.

Das erfindungsgemäße Verfahren eignet sich insbesondere zur Auftrennung von Gemischen von gegebenenfalls Heteroatome aus der Gruppe Stickstoff und Sauerstoff enthaltenden Perfluorkohlenwasserstoffen, welche als Verbindungen B gegebenenfalls Heteroatome enthaltende Perfluorkohlenwasserstoffe mit cyclischer Struktur und als Verbindungen A gegebenenfalls Heteroatome enthaltende cyclische Perfluorkohlenwasserstoffe, welche Reste mit unverzweigter acyclischer Struktur enthalten, enthalten. Als Perfluorkohlenwasserstoffe oder Perfluorcarbone werden im Rahmen der vorliegenden Anmeldung gesättigte oder aromatische, insbesondere gesättigte, perfluorierte organische Verbindungen bezeichnet, welche aus Kohlenstoff und Fluor bestehen und gegebenenfalls noch Heteroatome, beispielsweise Stickstoff, Sauerstoff oder Schwefel, insbesondere Stickstoff oder Sauerstoff, in der Kohlenstoffkette enthalten können. Die Verbindungen können beispielsweise bis zu 20, insbesondere 5-20, Kohlenstoff-Atome enthalten. Als Perfluorkohlenwasserstoffe vom Typ der Verbindungen B kommen beispielsweise mono- bis tetracyclische Verbindungen mit 3-18, insbesondere 6-12 Ringgliedern in Frage. Es kann sich um Verbindungen mit anellierten Ringsystemen, beispielsweise Decalin, oder mit diamantoiden Ringsystemen, beispielsweise Adamantan, oder um Verbindungen, in welchen zwei 5- und/oder 6-gliedrige Ringe miteinander, gegebenenfalls durch eine niedere Alkylenkette, verbunden sind, beispielsweise Cyclohexylmorpholin oder 1,3-Dipiperidinopropan, handeln. Das Verfahren eignet sich in gleicher Weise auch zur Auftrennung von Gemischen solcher fluorierter Verbindungen A und B, welche noch einen Rest an Wasserstoffatomen enthalten, also nur partiell fluoriert sind oder welche durch weitere Halogenatome substituiert sind. Das Verfahren eignet sich insbesondere zur Auftrennung solcher Gemische, welche eine cyclische Verbindung B enthalten und durch Fluorierung, insbesondere elektrochemische Fluorierung der der cyclischen Verbindung B entsprechenden nichtfluorierten Ausgangsverbindung erhalten wurden. Beispielsweise eignet sich das Verfahren zur Auftrennung von Gemischen, welche cyclische Verbindungen B, insbesondere gegebenenfalls Heteroatome enthaltende Perfluorkohlenwasserstoffe mit cyclischer Struktur, und dazu isomere cyclische Verbindungen A, welche Reste mit unverzweigter acyclischer Struktur enthalten, enthalten. Ebenfalls eignet sich das Verfahren zur Auftrennung von Gemischen, welche Verbindungen der Formel B, insbesondere cyclische gegebenenfalls Heteroatome enthaltende Perfluorkohlenwasserstoffe mit cyclischer Struktur, und dazu homologe durch niedere unverzweigte Perfluoralkylreste, insbesondere Trifluormethylreste, substuierte cyclische Verbindungen A enthalten.

Die kritischen Moleküldurchmesser von Verbindungen sind berechenbar, beispielsweise unter Benutzung der van-der-Waalschen Atomradien (vgl. O. Grubner, et al, Molekularsiebe, VEB Deutscher Verlag der Wissenschaften, Berlin 1968, Seiten 63, 64). Der kritische Durchmesser von kugelförmigen Molekeln (z.B. Edelgase) ist gleich dem Durchmesser einer diese Molekel beschreibenden Kugel. Bei symmetrischen, tetraedrisch angeordneten Molekeln (z.B. CCl₄) gleicht der kritische Durchmesser dem Durchmesser des Kreises, der die dreieckige Tetraederwand beschreibt.

Bei symmetrischen oktaedrisch angeordneten Molekeln (z.B. SF₆) gleicht der kritische Durchmesser dem Durchmesser des Kreises, der die quadratförmige Basis des Oktaeders wiedergibt. Bei zweiatomigen Molekeln ist ihr kritischer Durchmesser durch den Durchmesser des Kreises, der diese Molekeln in der senkrechten Ebene zu ihrer Länge beschreibt, gegeben. Die kritischen Durchmesser von n-Paraffinen, deren Molekeln lineare ("Zickzack"-) Anordnung besitzen, sind durch den Durchmesser des größten Kreises, welcher in der zur Paraffinkette senkrecht liegenden Ebene die Molekeln beschreibt, gegeben und sind bei allen n-Paraffinen dieselben. Analoges gilt auch für acyclische Perfluorkohlenwasserstoffverbindungen bzw. -reste.

Als Molekularsiebe für das erfindungsgemäße Verfahren eignen sich insbesondere anorganische Molekularsiebe, z.B. Aluminosilicate oder Silicalite. Als zweckmäßig erweisen sich natürliche und künstliche Zeolithe, insbesondere Zeolithe der Typen A und X. Je nach dem kritischen Moleküldurchmesser der unverzweigten, acyclischen Reste der Verbindungen A kann die Porengröße der eingesetzten Molekularsiebe variieren. Im allgemeinen werden gute Trenneffekte mit Molekularsieben mit mittleren Porenöffnungsdurchmessern von 4,5 bis 6,5 Angström, insbesondere 5 bis 6,5, vorzugsweise 5-6 Angström, erzielt. Die Molekularsiebe können als Partikel unterschiedlicher Form eingesetzt werden, z.B. in Form von Granulaten oder Perlen.

Gemische von gegebenenfalls Heteroatome enthaltenden cyclischen Perfluorkohlenwasserstoffen mit unverzweigte acyclische Reste enthaltenden perfluorierten Nebenprodukten, welche nach dem erfindungsgemäßen Verfahren aufgetrennt werden können, können beispielsweise erhalten werden, indem man entsprechende nichtfluorierte cyclische Verbindungen nach an sich bekannten Methoden fluoriert. So können die nichtfluorierten Ausgangsverbindungen durch elektrochemische Fluorierung fluoriert werden, indem man Lösungen der Verbindungen in flüssigem Fluorwasserstoff elektrolysiert. Zweckmäßig werden hierfür Lösungen von 4-30, vorzugsweise 5-10 Gew.-%, einer nicht fluorierten Ausgangsverbindung in flüssigem Fluorwasserstoff eingesetzt. Die Elektrolyse findet vorteilhaft bei Temperaturen zwischen -25 und + 10 °C, vorzugsweise -5 und + 5 °C, einer Anodenstromdichte von 2-30 mA/cm² und einer Zellspannung von 3-10, insbesondere 4-8 V, statt. Zur weiteren Aufarbeitung wird das sich als schwere Phase am Boden der verwendeten Elektrolysezelle absetzende rohe Reaktionsprodukt abgetrennt und zur Zersetzung von allfälligen nur teilfluorierten Nebenprodukten einer Behandlung mit einer Alkali- oder Erdalkalimetallbase, insbesondere einem Alkali- oder Erdalkalimetallhydroxid, in Gegenwart von Wasser und ggf. einem niederen aliphatischen primären oder sekundären Amin bei einer zur Zersetzung von nur partiell fluorierten Nebenprodukten ausreichend hohen Temperatur unterworfen. Diese Verfahrensstufe kann nach an sich bekannten Methoden erfolgen. So kann das Reaktionsgemisch beispielsweise für eine Dauer von mehreren Stunden bis zu 8 Tagen unter Rückfluß zum Sieden erhitzt werden. Aus dem entstandenen Reaktionsgemisch können die perfluorierten Verbindungen durch fraktionierte Destillation isoliert werden. Die durch fraktionierte Destillation aus dem Reaktionsgemisch abgetrennten Produkte sind im allgemeinen frei von nicht perfluorierten Produkten. Sie stellen im allgemeinen jedoch Gemische dar, welche neben dem perfluorierten cyclischen Hauptprodukt noch solche perfluorierten Nebenprodukte enthalten, welche zu dem Hauptprodukt isomer sind oder ein ähnliches Molekulargewicht wie das Hauptprodukt besitzen und acyclische Reste enthalten können. Beispielsweise findet in piperidinringhaltigen Verbindungen unter den Bedingungen der elektrochemischen Perfluorierung teilweise in den Piperidinringen eine Ringverengung statt, so daß neben den perfluorierten Piperidinverbindungen in geringerem Maße auch dazu isomere Methylpyrrolidin-Verhindungen entstehen. Derartige Isomerengemische können beispielsweise nach dem erfindungsgemäßen Verfahren getrennt werden.

Erfindungsgemäß kann die Kontaktierung des aufzutrennenden Gemisches mit dem Molekularsieb in flüssiger Phase oder in der Gasphase durchgeführt werden. Die Adsorption in flüssiger Phase kann bei Temperaturen zwischen Raumtemperatur und Siedetemperatur des aufzutrennenden Gemisches durchgeführt werden. Beispielsweise können aufzutrennende Gemische von bei Raumtemperatur flüssigen Verbindungen oder Lösungen von Gemischen von bei Raumtemperatur wachsartig bis festen Verbindungen in einen das Molekularsieb enthaltenden Reaktor eingeführt und darin für eine zur Adsorption der geradkettige acyclische Reste enthaltenden Verbindungen A ausreichende Zeit mit dem Molekularsieb in Kontakt gehalten werden. Als Lösungsmittel eignen sich insbesondere flüssige Perfluorcarbone, beispielsweise Perfluorodecalin. Zweckmäßig wird für eine gute Durchmischung des Gemisches mit den Molekularsiebpartikeln beispielsweise durch Schütteln oder Rühren gesorgt. Hierbei kann die Adsorption von Verbindungen A an das Molekularsieb bei Raumtemperatur oder erhöhten Temperaturen unterhalb des Siedepunktes des Reaktionsgemisches erfolgen. Die einzusetzende Menge an Molekularsieb kann je nach Art des Molekularsiebs und Art der zu adsorbierenden Verbindung A variieren. Beispielsweise können das 2-50-fache des Gewichtes an zu adsorbierender Verbindung A eingesetzt werden. Der Kontakt des Molekularsiebes mit dem Aufzutrennenden Gemisch kann auch in der Gasphase derart erfolgen, daß das Gemisch gasförmig durch einen mit Molekularsiebpartikeln gefüllten Reaktor geleitet wird. Beispielsweise kann das Gemisch über einen Verdampfer zusammen mit einem Trägergas durch den das Molekularsieb enthaltenden Reaktor mit einer solchen Strömungsgeschwindigkeit geleitet werden, daß die Kontaktzeit mit dem Molekularsieb zur Adsorption der Verbindung der Formel A ausreicht. Die Adsorption aus der Gasphase kann bei Temperaturen zwischen 50 und 300 °C und bei Drucken von 0,1 bis 10 bar erfolgen.

Nach erfolgter Adsorption der Verbindungen A wird das mit Verbindung A beladene Moleklarsieb von den nicht adsorbierten Verbindungen B abgetrennt. Nach einer Gasphasenadsorption kann dies durch Nachspülen mit Trägergas erfolgen. Wenn die Adsorption aus flüssiger Phase erfolgte, können die mit Verbindungen A beladenen Molekularsiebpartikel auf an sich bekannte Weise durch Filtieren oder Dekantieren von nicht adsorbierter Flüssigkeit abgetrennt werden. Je nach Menge und Raumbedarf der adsorbierten Moleküle der Verbindung A können die Molekularsiebpartikel auch noch mit geringen Mengen von beispielsweise äußerlich als Film anhaftenden Molekülen der Verbindung B beladen sein.

Die anschließende Desorption der Verbindungen der Formel A von den Molekularsiebpartikeln kann auf an sich bekannte Weise beispielsweise durch Verdrängungsdesorption oder durch thermisches Austreiben erfolgen. Eine Verdrängungsdesorption kann beispielsweise auf an sich bekannte Weise durch Behandeln des mit Verbindungen A beladenen Molekularsiebs mit Wasser erfolgen. Falls die Molekularsiebpartikel auch noch geringe Anteile an Verbindungen B enthalten, empfiehlt sich eine thermische Desorption. Hierbei können zunächst äußerlich anhaftende Verbindungen B abgedampft und anschließend durch weiteres Erhitzen die adsorbierten Verbindungen A desorbiert werden. Die thermische Desorption kann durch Erhöhung der Temperatur auf einen Temperaturbereich zwischen Siedepunkt und Zersetzungstemperatur der zu desorbierenden Verbindungen erfolgen. Zweckmäßig liegt die Desoprtionstemperatur ca. 100-150 °C oberhalb der Adsorptionstemperatur in der Gasphase. Sofern das aufzutrennende Gemisch mehrere adsorbierbare Verbindungen A enthält, kann bei der Desorption ebenfalls ein Gemisch dieser Verbindungen A erhalten werden. Derartige Gemicche von Verbindungen A können gewünschtenfalls auf an sich bekannte Weise chromatographisch, z.B. durch Dünnschicht- oder Gaschromatographie, weiter aufgetrennt werden.

Nach dem erfindungsgemäßen Verfahren werden Molekularsiebe eingesetzt, deren Porenöffnungsdurchmesser bis zu 30 % unter dem kritischen Moleküldurchmesser der zu adsorbierenden Verbindungen A liegen. Da die Porenöffnungsdurchmesser der eingesetzten Molekularsiebe somit kleiner sind als die kritischen Moleküldurchmes3er aller in dem aufzutrennenden Gemisch enthaltenen Verbindungen, ist es überraschend, daß trotzdem eine Trennung der Verbindungen A von den Verbindungen B durch Adsorption an Molekularsieben, beispielsweise Zeolithen, nach dem erfindungsgemäßen Verfahren möglich ist.

Die nachstehenden Beispiele sollen die Erfindung näher erläutern, ohne jedoch ihren Umfang zu beschränken.

### Beispiel 1:

Auftrennung eines Gemisches aus Perfluordecalin und Perfluormethyldecalin

10 g eines zeolithischen Molekularsiebs mit einem mittleren Porenöffnungsdurchmesser von 0,5 nm (Hersteller Fa. Merck) wurden durch 15-stündiges Tempern bei 500 °C im Muffelofen aktiviert. Die so vorbehandelten Molekularsiebpartikel wurden in einem Glaskolben mit einem Gemisch von 4 g Perfluordecalin und 4 g Perfluormethyldecalin versetzt und zwei Stunden bei Raumtemperatur geschüttelt. Anschließend wurde die nichtadsorbierte Flüssigkeit vom Molekularsieb abgetennt. Die nicht adsorbierte Phase enthielt ca. 4 g reines Perfluordecalin. Zur Desorption der adsorbierten Verbindung wurden die Molekularsiebpartikel in dem Glaskolben während zwei Stunden bei Raumtemperatur mit Wasser geschüttelt. Das desorbierte Produkt wurde vom Wasser durch Destillation in eine gekühlte Vorlage abgetrennt und gaschromatographisch und über ¹⁹ F-NMR-Spektroskopie als Perfluormethyldecalin identifiziert. Die Ausbeute an Perfluormethyldecalin betrug ca. 3,5 g.

### Beispiel 2:

Auftrennung eines Gemisches aus Perfluortrimethyladamantan und Perfluoradamantan

0,5 g Perfluortrimethyladamantan und 0,5 g Perfluoradamantan wurden in 15 g Perfluordecalin gelö3t. Die Lösung wurde mit 20 g des in Beispiel 1 verwendeten aktivierten Molekularsiebs (Porenöffnungsdurchmesser 0,5 nm) 4 Stunden bei einer Temperatur von 30-40 °C geschüttelt. Anschließend wurde die nicht adsorbierte Flüssigkeit von den Molekularsiebteilchen abgetrennt. Diese Flüssigkeit enthielt die eingewogene Menge Perfluoradamantan und Perfluordecalin. Die Desorption des adsorbierten Anteils des Ausgangsgemisches erfolgte durch Aufheizen des Molekularsiebs auf 180 °C unter vermindertem Druck (ölpumpenvakuum) und Auffangen des verflüchtigten Produktes in einer gekühlten Vorlage. Es wurden so ca. 0,3 g Perfluortrimethyladamantan erhalten, welches durch ¹⁹F-NMR-Spektroskopie und Massenspektrometrie identifiziert wurde.

### Beispiel 3:

Auftrennung eines Gemisches aus Perfluorcyclohexylmorpholin und Perfluor-n-hexylmorpholin
A) Herstellung des Gemisches:
   Eine 5- bis 15-%ige Lösung von Morpholincyclohexen-(1) in vorgetrocknetem gekühltem flüssigem Fluorwasserstoff wurde in einer Elektrolysezelle bei einer Anodenstromdichte von 3-20/mA cm² , einer Zellspannung von 5 bis 6,5 V und einer Zelltemperatur von -8 bis + 5° C perfluoriert. Von Zeit zu Zeit wurden weiteres in flüssigem Fluorwasserstoff gelöstes Morpholincyclohexen-(1) und verbrauchter Fluorwasserstoff nachgefüllt, um ein kontinuierliches Arbeiten der Zelle zu ermöglichen. Die am Zellboden sich sammelnde, das rohe Reaktionsprodukt enthaltende schwere Phase wurde von Zeit zu Zeit abgelassen. Das Rohprodukt wurde mit jeweils den gleichen Volumina einer wäßrigen 8-N Kaliumhydroxidlösung und Dibutylamin versetzt. Das Gemisch wurde 8 Tage am Rückfluß erhitzt. Anschließend wurde das Gemisch fraktionierend destilliert. Bei der Destillation erhielt man eine Hauptfraktion im Siedebereich von 145 -148 C. Die gaschromatographische Analyse ergab, daß das Destillat ein Gemisch aus 65 % Perfluorocyclohexylmorpholin, 33 % Perfluoro-n-hexylmorpholin und 2 % weiterer Nebenprodukte darstellte.
B) Auftrennung des unter A) erhaltenen Gemisches:
   Auf eine mit einem zeolithischen Molekularsieb der Porengröße 5 Angström gefüllte Glassäule, durch die ein konstanter Strom Helium mit einer Strömungsgeschwindigkeit von 60 ml/Minute floß, wurde bei 120 °C über einen Verdampfer das vorstehend erhaltene Gemisch aus Perfluorcyclohexylmorpholin und Perfluor-n-hexylmorpholin aufgegeben. Während das Perfluorcyclohexylmorpholin die Säule passierte, wurde das den C₆F₁₃-Rest enthaltende Nebenprodukt zurückgehalten. Es wurde so perfluoriertes Cyclohexylmorpholin mit einem Siedepunkt von 147,5 bis 149,5 °C gewonnen. Nach dem alles Perfluorcyclohexylmorpholin und überschüssiges Perfluor-n-hexylmorpholin die Säule verlassen hatten, wurde die Temperatur auf 300 °C gesteigert. In einer Kühlfalle am Ausgang der Säule kondensierte reines Perfluor-n-hexylmorpholin mit einem Siedepunkt von 149 bis 150 °C.

### Beispiel 4:

Auftrennung eines Gemisches aus perfluoriertem 1,3-Dipiperidinopropan und den dazu isomeren perfluorierten 1-(3-Methylpyrrolidino)-3-piperidionopropan und 1,3-Di(3-methylpyrolidino)-propan.
A) Herstellung des Gemisches:
   Eine 10%ige Lösung von 1,3-Dipiperidinopropan in vorgetrocknetem gekühltem flüssigem Fluorwasserstoff wurde in einer Elektrolysezelle bei einer Temperatur von +2 °C, einer Anodenstromdichte von 2,5 bis 25 mA/cm² und einer Zellspannung von 4,5 bis 7,0 V perfluoriert. Von Zeit zu Zeit wurden in flüssigem Fluorwasserstoff gelöstes Dipiperidinopropan und verbrauchter Fluorwasserstoff nachgefüllt, um ein kontinuierliches Arbeiten der Zelle zu ermöglichen. Das sich am Zellboden sammelnde rohe Reaktionsprodukt wurde von Zeit zu Zeit abgelassen und, wie in Beispiel 3 A) beschrieben, aufgearbeitet. Bei der Destillation wurde eine Hauptfraktion erhalten, welche bei 195 bis 203 °C siedete. Eine gaschromatographische Auftrennung des Gemisches ergab, daß dieses 58 % perfluoriertes 1,3-Dipiperidinopropan, 36 % perfluoriertes 1-(3-Methylpyrrolidino)-3-piperidinopropan und 6 % perfluoriertes 1,3-Di(3-methylpyrrolidino)-propan enthielt.
B) Auftrennung des vorstehend erhaltenen Gemisches Die Trennung wurde mittels temperaturprogrammierter Gaschromatographie auf einem präparativen Gaschromatographen durchgeführt. Das Gemisch wurde über eine mit einem Zeolith-Molekularsieb mit einer Porengröße von 5 A gefüllte gaschromatographische Trennsäule (Glassäule 1,7 mm x 3 mm Innendurchmesser) bei ca. 180 °C geleitet. Während das perfluorierte 1,3-Dipiperidinopropan die Säule passierte, wurden die beiden einen Trifluormethylrest enthaltenden Nebenprodukte weitgehend zurückgehalten. Es wurde so reines perfluoriertes 1,3-Dipiperidinopropan mit einem Siedepunkt von 191 °C gewonnen. Nachdem alles perfluorierte 1,3-Dipiperidinopropan die Säule verlassen hatte, wurde durch thermische Desorption bei einer um 100 °C erhöhten Temperatur ein Gemisch aus perfluoriertem 1-(3-Methylpyrrolidino)-3-piperidinopropan und perfluoriertem 1,3-Di(3-methylpyrrolidino)-propan gewonnen. Anschließend wurde die Trennung der beiden Trifluormethylreste enthaltenden Verbindungen durch präparative Gaschromatographie an einer festen Phase aus Methylsiliconöl an anorganischem Trägermaterial (=SE-30, Hersteller Chrompack) durchgeführt. Es wurden perfluoriertes 1-(3-Methylpyrrolidino)-3-piperidinopropan mit einem Siedepunkt von 189 °C und 1,3-Di(3-methylpyrrolidino)-propan mit einem Siedepunkt von 186 °C erhalten.

## Patentansprüche

1. Verfahren zur Auftrennung von Gemischen partiell- oder perfluorierter Kohlenwasserstoffverbindungen, welche gegebenenfalls noch Kettenglieder aus der Gruppe der Heteroatome Stickstoff, Sauerstoff, Bor, Phosphor und Schwefel enthalten und/oder durch weitere Halogenatome substituiert sein können, dadurch gekennzeichnet, daß man solche partiell-oder perfluorierten cyclischen Verbindungen, welche durch Reste mit unverzweigter acyclischer Struktur und mit einem kritischen Moleküldurchmesser im Bereich von 4 bis 7 Angström substituiert sind, und welche nachfolgend als Verbindungen A bezeichnet werden, von solchen partiell- oder perfluorierten cyclischen Verbindungen, deren kritischer Moleküldurchmesser mindestens das 1,1-fache desjenigen der vorgenannten unverzweigten acyclischen Reste beträgt, und welche keine unverzweigten acyclischen Reste besitzen, und auch nicht durch andere Reste substituiert sind, deren kritischer Moleküldurchmesser nicht mindestens das 1,1-fache desjenigen der vorgenannten unverzweigten acyclischen Reste beträgt, und welche nachfolgend als Verbindungen B bezeichnet werden, in flüssiger oder gasförmiger Phase trennt, indem man
a) das Gemisch oder eine Lösung des Gemisches in einem inerten organischen Lösungsmittel, dessen Moleküldurchmesser gröBer als derjenige der vorgenannten unverzweigten acyclischen Reste ist, mit einer zur Adsorption der Verbindungen A ausreichenden Menge eines Molekularsiebs kontaktiert, dessen mittlerer Porenöffnungsdurchmesser im Bereich des kritischen Moleküldurchmessers der unverzweigten acyclischen geradekettigen Reste der Verbindungen A oder bis zu 30 % darunter liegt,
b) das überwiegend mit Verbindungen A beladene Molekularsieb von der an Verbindungen A abgereicherten, die Verbindungen B enthaltenden flüssigen oder gasförmigen Phase abtrennt, und
c) die an dem Molekularsieb adsorbierten Verbindungen desorbiert.

2. Verfahren nach Anspruch 1 dadurch gekennzeichnet, daß als aufzutrennende Gemische Gemische eingesetzt werden, welche als Verbindungen B gegebenenfalls Heteroatome aus der Gruppe Stickstoff und Sauerstoff enthaltende Perfluorkohlenwasserstoffe mit cyclischer Struktur und als Verbindungen A gegebenenfalls Heteroatome enthaltende cyclische Perfluorkohlenwasserstoffe, welche Reste mit unverzweigter acyclischer Struktur enthalten, enthalten.

3. Verfahren nach Anspruch 1 oder 2, dadurch gekennzeichnet, daß als aufzutrennende Gemische Gemische eingesetzt werden, welche eine cyclische Verbindung B enthalten und durch Fluorierung, insbesondere elektrochemische Fluorierung, der der Verbindung B entsprechenden nichtfluorierten cyclischen Ausgangsverbindung erhalten werden.

4. Verfahren nach Anspruch 1-3, dadurch gekennzeichnet, daß als aufzutrennende Gemische Gemische eingesetzt werden, welche cyclische Verbindungen B und dazu isomere cyclische Verbindungen A, welche Reste mit unverzweigter acyclischer Struktur enthalten, enthalten.

5. Verfahren nach Anspruch 1-3 dadurch gekennzeichnet, daß als aufzutrennende Gemische Gemische eingesetzt werden, welche Verbindungen B und dazu homologe, durch niedere unverzweigte Perfluoralkylreste, insbesondere Trifluormethylreste, substituierte Verbindungen A enthalten.

6. Verfahren nach Anspruch 1 dadurch gekennzeichnet, daß anorganische Molekularsiebe eingesetzt werden.

7. Verfahren nach Anspruch 6 dadurch gekennzeichnet, daß als Molekularsiebe natürliche oder synthetische Zeolithe eingesetzt werden.

8. Verfahren nach Anspruch 6 oder 7 dadurch gekennzeichnet, daß Molekularsiebe mit einem mittleren Porenöffnungsdurchmesser von 5-6,5 Angström eingesetzt werden.

## Claims

1. A process for separating mixtures of partially fluorinated or perfluorinated hydrocarbon compounds which optionally additionally contain chain members from the group of the heteroatoms nitrogen, oxygen, boron, phosphorus and sulphur and/or may be substituted by further halogen atoms, characterised in that such partially fluorinated or perfluorinated cyclic compounds which are substituted by residues having an unbranched, acyclic structure and having a critical molecular diameter in the range of 4 to 7 angstroms, and which will be designated below as compounds A, are separated from such partially fluorinated or perfluorinated cyclic compounds the critical molecular diameter of which is at least 1.1 times that of the afore-mentioned unbranched acyclic residues and which do not have any unbranched acyclic residues and are also not substituted by other residues, the critical molecular diameter of which is not at least 1.1 times that of the afore-mentioned unbranched acyclic residues, and which will be designated below as compounds B, in liquid or gaseous phase, in that
a) the mixture or a solution of the mixture in an inert organic solvent, the molecular diameter of which is greater than that of the aforementioned unbranched acyclic residues is contacted with a quantity of a molecular sieve sufficient for adsorption of the compounds A said molecular sieve having a mean open pore diameter in the range of between the critical molecular diameter of the unbranched acyclic straight-chained residue of the compounds A and 30 % therebelow,
b) the molecular sieve which is laden predominantly with compounds A is seperated from the liquid or gaseous phase which contains the compounds B and which is improverished in terms of compounds A, and
c) the compounds adsorbed on the molecular sieve are desorbed.

2. A process according to Claim 1, characterised in that mixtures which contain as compounds B perfluorohydrocarbons having a cyclic structure and optionally containing heteroatoms from the group nitrogen and oxygen, and as compounds A cyclic perfluorohydrocarbons optionally containing heteroatoms which contain residues having an unbranched acyclic structure, are used as the mixtures to be separated.

3. A process according to Claim 1 or 2, characterised in that mixtures which contain a cyclic compound B and are obtained by fluorination, in particular by electrochemical fluorination, of the non-fluorinated cyclic starting compound corresponding to sponding to compound B are used as the mixtures to be seperated.

4. A process according to Claims 1 to 3, characterised in that mixtures which contain cyclic compounds B and cyclic compounds A which are isomeric thereto and which contain radicals with an unbranched acyclic structure are used as the mixtures to be seperated.

5. A process according to Claims 1 to 3, characterised in that mixtures which contain compounds B and compounds A homologous thereto which are substituted by lower, unbranched perfluoroalkyl radicals, in particular trifluoromethyl radicals, are used as the mixtures to be separated.

6. A process according to Claim 1, characterised in that inorganic molecular sieves are used.

7. A process according to Claim 6, characterised in that natural or synthetic zeolites are used as molecular sieves.

8. A process according to Claim 6 or 7, characterised in that molecular sieves having a mean open pore diameter of 5 - 6.5 angstroms are used.

## Revendications

1. Procédé de séparation de mélanges de composés hydrocarbonés partiellement fluorés ou perfluorés qui contiennent éventuellement encore des chaînons du groupe des hétéroatomes azote, oxygène, bore, phosphore et soufre et/ou peuvent être substitués par des atomes d'halogène supplémentaires, caractérisé en ce qu'on sépare en phase liquide ou gazeuse de tels composés cycliques partiellement fluorés ou perfluroés qui sont substitués par des restes possédant une structure acyclique non ramifiée et un diamètre de molécule critique dans le domaine de 4 à 7 Angströms et qui sont désignés dans ce qui suit par composés A, de tels composés cycliques partiellement fluorés ou perfluorés dont le diamètre de molécule critique s'élève à au moins 1,1 fois celui des restes acycliques non ramifiés précités et qui ne possèdent pas de restes acycliques non ramifiés précités et qui ne sont pas substitués non plus par d'autres restes dont le diamètre de molécule critique ne s'élève pas à au moins 1,1 fois celui des restes acycliques non ramifiés précités et qui sont désignés dans ce qui suit par composés B,
a) en mettant en contact le mélange ou une solution du mélange dans un solvant organique inerte dont le diamètre de molécule est supérieur à celui des restes acycliques non ramifiés précités avec une quantité suffisante d'un tamis moléculaire pour l'adsorption des composés A, tamis dont le diamètre moyen de pore se situe dans le domaine du diamètre de molécule critique des restes acycliques à chaîne droite non ramifiée des composés A ou jusqu'à 30 % au-dessous,
b) en séparant le tamis moléculaire essentiellement chargé de composés A de la phase liquide ou gazeuse renfermant les composés B, appauvrie en composés A et
c) en désorbant les composés adsorbés sur le tamis moléculaire.

2. Procédé selon la revendication 1, caractérisé en ce qu'on met en oeuvre comme mélanges à séparer des mélanges contenant, en tant que composés B, des hydrocarbures perfluorés de structure cyclique renfermant éventuellement des hétéroatomes du groupe de l'azote et de l'oxygène et, en tant que composés A, des hydrocarbures perfluorés cycliques contenant éventuellement des hétéroatomes qui renferment des restes avec une structure acyclique non ramifiée.

3. Procédé selon la revendication 1 ou 2, caractérisé en ce qu'on met en oeuvre comme mélanges à séparer des mélanges qui contiennent un composé B cyclique et sont obtenus par fluoration, notamment par fluoration électrochimique du composé de départ cyclique non fluoré correspondant au composé B.

4. Procédé selon les revendications 1 à 3, caractérisé en ce qu'on met en oeuvre comme mélanges à séparer des mélanges qui contiennent des composés B cycliques et en outre des composés A cycliques isomères comportant des restes avec une structure acyclique non ramifiée.

5. Procédé selon les revendications 1 à 3, caractérisé en qu'on met en oeuvre comme mélanges à séparer des mélanges contenant des composés B et en outre des composés A homologues substitués par des restes perfluoroalkyles non ramifiés, notamment des restes trifluorométhyle.

6. Procédé selon la revendication 1, caractérisé en ce qu'on met en oeuvre des tamis moléculaires minéraux.

7. Procédé selon la revendication 6, caractérisé en ce qu'on met en oeuvre, en tant que tamis moléculaires, des zéolithes naturelles ou synthétiques.

8. Procédé selon la revendication 6 ou 7, caractérisé en ce qu'on met en oeuvre des tamis moléculaires ayant un diamètre moyen de pore de 5-6,5 Angströms.
